# EUROPÄISCHE PATENTANMELDUNG

(11) **EP 2 510 912 A1**
(43) Veröffentlichungstag der Anmeldung: **17.10.2012**
(21) Anmeldenummer: 12001738.9
(22) Anmeldetag: 14.03.2012
(51) Int. Cl.: A61F 13/15, A61L 15/20

(54) **Medizinische Hautabdeckung zur Behandlung von Infektionen der Haut**

(30) Priorität: 14.03.2011 DE 102011013920
(71) Anmelder: Winkelmann, Wolfgang, 72076 Tübingen (DE)
(72) Erfinder: Winkelmann, Wolfgang, 72076 Tübingen (DE)
(74) Vertreter: Wüstefeld, Regine Marie

(57) **Zusammenfassung**

Die Erfindung betrifft eine medizinische Hautabdeckung zur Behandlung und Verhinderung von Infektionen der Haut, mit einer Hautauflage (3) und einer Deckschicht (5), wobei die Hautauflage (3) mit einem antiphlogistischen und/oder antibakteriellen und/oder antiviralen und/oder antimykotischen Mittel versehen ist, das ozonisiertes Pflanzenöl und/oder dessen Bestandteile umfaßt.

Die medizinische Hautabdeckung kann eine Slip-Einlage, eine Windel oder ein Teil davon, ein desinfizierendes Unterlegvlies oder ein Pflaster sein. Sie kann verwendet werden zur Herstellung eines Präparats für die Behandlung von entzündlichen Prozessen der Haut, von Furunkeln und Abszessen, Akne und Pilzinfektionen, "ulcus cruris" und diabetischem Fuß. Sie kann auch zur Herstellung eines Zeckenpflasters dienen.

## Beschreibung

Die Erfindung betrifft eine medizinische Hautabdeckung zur Behandlung von Infektionen der Haut, mit einer Hautauflage und einer Deckschicht, die auf der der Haut zugewandten Seite wahlweise zumindest teilweise eine Klebeschicht aufweist. Die medizinische Hautabdeckung ist unter anderem zur Behandlung von Furunkeln und Abszessen, Akne und Pilzinfektionen geeignet.

Die Haut ist ein wichtiges Organ des Menschen, die vor der Außenwelt schützt und somit auch allen Umwelteinflüssen ausgesetzt ist. Infektionen der Haut (Cutis) mit den unterschiedlichsten Ursachen gibt es in vielfältiger Art. Zum einen ist hier die Gruppe der Entzündungen zu nennen. Zumeist sind solche Entzündungen auf die oberen Schichten der Haut, die Oberhaut (Epidermis) und die Lederhaut (Dermis oder Corium) beschränkt. Hautentzündungen sind unter den Begriffen Dermatitis, entzündliche Dermatosen oder Ekzeme bekannt. Als Ursachen solcher Hautentzündungen können mikrobielle Noxen, Allergien, aber auch ganz einfach chemische oder physikalische Reize gesehen werden. Die Haut reagiert zumeist mit einer Rötung, die je nach Fortschritt der Entzündung mit einer mehr oder weniger starken Schwellung einhergeht. Es können sich auch oder nur Bläschen bilden. Auch eine Krustenbildung kann Folge einer Hautentzündung sein. Ein Teil solcher Hautentzündungen kann durch Staphylokokken oder Streptokokken ausgelöst werden und ist insofern auch nicht mehr als harmlos zu bezeichnen. Andere Hauterkrankungen können auf einer Infektion mit Viren der Herpes-Gruppe, wie dem Herpes-simplex-Virus, beruhen.

Bei harmloseren Hauterkrankungen ist es bekannt, Tinkturen, Essenzen und Salben auf der Basis von Kamille und Calendula einzusetzen. Bei Hautentzündungen bedingt durch Staphylokokken oder Streptokokken müssen bisher Antibiotikasalben wie Fucidine® verwendet werden. Die Salben müssen zu ihrer Wirksamkeit auf der Hautfläche, auf die sie aufgetragen wurden, gehalten werden. Je nach Größe der Fläche dienen hierzu Verbände oder Pflaster.

Eine andere Gruppe von Infektionen betrifft die durch Pilze hervorgerufenen Hautveränderungen. Hier ist besonders der Fußpilz zu nennen, der im übrigen oft mit Entzündungen der Haut einhergeht. Liegt eine solche kombinierte Infektion vor, ist es bisher in der Regel üblich, zunächst die Entzündung zu behandeln, bevor der Pilz bekämpft wird. Bei der Behandlung des Pilzes selbst wird in der Regel ein Antimykotikum eingesetzt. Auf diese Weise ist die Behandlung oft langwierig und die eingesetzten Mittel sind nicht unproblematisch.

Um dermal zu applizierende Wirkstoffe mit der Hautoberfläche in Kontakt zu bringen, sind medizinische Hautabdeckungen in Form von medizinischen und/oder therapeutischen Pflastern als sogenannte transdermale Pflaster oder transdermale Systeme bekannt. Insbesondere bekannt sind sogenannte Rheumapflaster mit verschiedenen und/oder unterschiedlichen Wirkstoffen, welche an dem jeweiligen schmerzenden Körperteil aufgelegt und durch Kleben befestigt werden. Aber auch die Behandlung im Rahmen koronarer Herzerkrankungen und ihren Begleiterscheinungen, wie Angina Pectoris, wird u.a. über Nitroglycerinhaltige medizinische Pflaster durchgeführt. Noch ein anderer Einsatzbereich betrifft die Behandlung von Kinetosen (Reisekrankheit) mit den Wirkstoff Scopolamin aufweisenden Pflastern.

Im Rahmen der vorliegenden Erfindung ist der Begriff des medizinischen Pflasters weit zu verstehen, so daß er ebenso ein für therapeutische Zwecke verwendetes Pflaster umfaßt. Hier ist z.B. das Nikotinpflaster als bekannt zu nennen, welches Raucher bei einer Entwöhnung unterstützen soll. Weitere bekannte Pflaster sind z.B. solche, die zur Behandlung von schmerzhaften Hühneraugen verwendet werden. Bei diesen Pflastern befindet sich der Wirkstoff Salicylsäure in einer Kammer, die aufgrund der typischerweise vorgegebenen Form solcher Hühneraugen kreisförmig ausgebildet ist. Das Pflaster wird über einen Zeitraum auf dem Hühnerauge belassen, um dieses durch den genannten Wirkstoff zu erweichen und herauslösbar zu machen.

Des weiteren ist in neuerer Zeit im Hinblick auf die bakteriell bedingte Borreliose als Folge eines Zeckenstiches die Entwicklung eines antibiotikumhaltigen Gels bekanntgeworden, das im Bereich der Einstichstelle auf die Haut aufgetragen wird. In Tierversuchen soll dieses auf die Haut aufgetragene antibiotikumhaltige Gel die Borreliose verursachenden Bakterien abtöten. Versuche am Menschen sind erst in Planung, wobei als antibiotikumhaltiges Gel, das per Pflaster aufgetragen wird, sowohl in den Tierversuchen als auch beim Menschen Azithromycin verwendet und speziell gegen Lyme-Borreliose eingesetzt wird.

Auch bei einem Zeckenbiß handelt es sich somit genau genommen nicht um einen Biß, sondern um einen Stich. Die befallene Hautstelle wird von der Zecke mit ihren Kieferklauen angeritzt, wobei sie zunächst ihr Hypostom, eine Art Stachel, in der Wunde verankert.

Zecken gehören in die Gruppe der Spinnentiere. Es ist bekannt, daß Zecken eine Vielzahl von Krankheiten übertragen können. Darunter sind die bekanntesten Erkrankungen die Frühsommermeningoenzephalitis (FSME) über den FSME-Virus und verschiedenste Borreliosen durch die unter die Gruppe der Borrelien fallenden Bakterien. Neben diesen hauptsächlich bekannten Erkrankungen wurde inzwischen eine Reihe weiterer, bakteriell bedingter Erkrankungen nachgewiesen.

Üblich ist bisher, zumindest in einem frühen Stadium einer bakteriell bedingten Erkrankung aufgrund eines "Zeckenbisses", eine Therapie mit einem Antibiotikum auf der Basis von Tetracyclinen, wie Doxycyclin, das intravenös oder oral verabreicht wird.

Ausgehend von diesem Stand der Technik lag der vorliegenden Erfindung daher die Aufgabe zugrunde, eine medizinische Hautabdeckung bereitzustellen, mit der harmlose Infektionen der Haut genauso wie Infektionen auf der Basis von Bakterien, Viren und Pilzen schonend, einfach, in einem Schritt behandelt werden können, mit der aber auch durch Tiere, insbesondere Gliederfüßer, wie beispielsweise Insekten und Spinnentiere, in den Körper eingeführte Infektionen auf der Basis von Bakterien und/oder Viren präventiv und/oder akut schonend, einfach und bei gleichzeitigem Befall von Viren und Bakterien in einem Schritt über die Haut behandelt werden können.

Gelöst wird diese Aufgabe durch eine medizinische Hautabdeckung, welche der Behandlung von Infektionen der Haut dienen kann, mit einer Hautauflage und einer Deckschicht, wobei die Hautauflage mit einem antiphlogistischen und/oder antibakteriellen und/oder antiviralen und/oder antimykotischen Mittel versehen ist, das ozonisiertes Pflanzenöl und/oder dessen Bestandteile umfaßt.

Die Deckschicht kann auf der der Haut zu- oder abgewandten Seite zumindest teilweise eine Klebeschicht aufweisen.

Das genannte, ozonisiertes Pflanzenöl und/oder dessen Bestandteile umfassende Mittel kann dabei auf der Hautauflage aufgetragen sein oder die Hautauflage ist mit dem Mittel imprägniert. Ozonisiertes Pflanzenöl, bzw. wahlweise dessen Bestandteile, hat dabei den Vorteil, in mehrfacher Hinsicht zu wirken. Grundsätzlich wird ihm zur gleichen Zeit antiphlogistische wie antibakterielle, antivirale und antimykotische Wirksamkeit zugeschrieben. Es ist aber bei der Anwendung wahrscheinlich, daß je nach Anwendungszweck nur ein Teil des möglichen Wirkungsspektrums benötigt wird. Es kann z.B. bei Fußpilzerkrankungen die antibakterielle wie antimykotische Wirksamkeit zugleich genutzt werden, während bei einer bakteriell bedingten Entzündung, wie z.B. dem sogenannten Grind bei Kindern, verschiedenen Akneformen oder sonstigen Hautentzündungen nur die antibakterielle Wirksamkeit zum Einsatz kommen wird.

Wie die umfangreichen Versuche übereinstimmend gezeigt haben, hat es außerdem eine schmerzlindernde bis schmerzstillende Wirkung, was ganz erstaunlich und überraschend war.

Der Anteil des Ozons in dem Pflanzenöl wird über die sogenannte Peroxidzahl definiert. Dabei liegt diese Peroxidzahl in der Regel über 200, vorzugsweise über 300 und kann auch noch weiter gesteigert werden.

Es ist sehr erstaunlich, daß ein in dieser Weise behandeltes Pflanzenöl auch nach langem Stehenlassen über Jahre hinweg einen angenehmen, eher frischen Geruch behält und nicht ranzig riecht, wie es eigentlich zu erwarten wäre.

Ozonisiertes Pflanzenöl in Form von ozonisiertem Olivenöl ist grundsätzlich bereits im Hinblick auf seine medizinische Anwendbarkeit bekannt. Es wird als solches, in Kapseln oder in Form von Salben angeboten. Als Anwendungsgebiete werden äußerliche Einreibungen mit dem ozonisierten Olivenöl bei Gelenkschmerzen und Rückenschmerzen, multipler Sklerose und auch bereits bei Akne genannt. Bei Neurodermitis und Psoriasis wird im Stand der Technik auch bereits eine kombinierte äußerliche Behandlung mit kolloidalem Silber vorgeschlagen, wobei beides nacheinander verwendet wird.

Es ist bisher aber nicht bekannt, die Eigenschaften des ozonisierten Olivenöls und/oder von dessen Bestandteilen in Verbindung mit einer medizinischen Hautabdeckung zu nutzen.

Damit die mit dem Pflanzenöl oder dessen Bestandteilen versehene Hautauflage in einfacher Weise an der Stelle fixiert werden kann, wo das Mittel wirken soll, kann des weiteren vorgesehen sein, daß die Hautauflage an der Klebeschicht befestigt ist. Dann ist sie bereits an der Klebeschicht befestigt und wird zusammen mit der Deckschicht als einteilige Hautabdeckung verwendet. Die Klebeschicht ist dann vorzugsweise ganzflächig auf der Deckschicht angebracht.

Gemäß der vorgenannten Ausführungsform der erfindungsgemäßen Hautabdeckung oder auch bei weiteren denkbaren Ausgestaltungen kann es sinnvoll sein, daß die Deckschicht eine größere Fläche aufweist als die Hautauflage. Sie überragt auf diese Weise die Hautauflage und dient nicht nur zu deren Fixierung, sondern gleichzeitig auch zu der Fixierung der Hautabdeckung insgesamt auf der Haut.

Gemäß einer weiteren Ausführungsform der erfindungsgemäßen medizinischen Hautabdeckung sind als ausgewählte erfindungsgemäße Pflanzenöle Rapsöl (auch Rüböl oder Rüpsenöl genannt), Sonnenblumenkernöl, Sojaöl, Rizinusöl, Olivenöl, Leinöl, Kokosöl, Palmöl, Teebaumöl oder zumindest ein Bestandteil, Derivat und/oder Mischungen davon zu nennen.

Es soll aber an dieser Stelle darauf hingewiesen werden, daß es sich dabei um eine exemplarische Aufzählung der gängigen Pflanzenöle handelt, und daß grundsätzlich jegliches Pflanzenöl für die Zwecke der vorliegenden Erfindung eingesetzt werden kann.

Das Derivat kann ein Ester sein, vorzugsweise ein Glycerinester einer mehrfach ungesättigten Fettsäure.

Als Bestandteil des Olivenöls sind insbesondere die Fettsäuren zu nennen. Hier kommt ganz bevorzugt die Ölsäure als einfach ungesättigte Fettsäure in Frage.

Es ist hinlänglich bekannt, daß die natürlichen pflanzlichen Öle Glycerinester der höheren geradzahligen Fettsäuren, d. h. der Glyceride darstellen und bis zu ca. 97% aus Triglyceriden, bis zu ca. 3% aus Diglyceriden und bis ca. 1% aus Monoglyceriden aufgebaut sind. Tri-, Di- und Monoglyceride bestehen jeweils aus einem Molekül Glycerin, verestert mit 3 Molekülen, 2 Molekülen bzw. 1 Molekül Fettsäure. Die chemischen, physikalischen und biologischen Eigenschaften der Pflanzenöle werden von der Art der Fettsäurekomponente und ihrer Verteilung über die Triglyceridmoleküle bestimmt. Es ist außerdem hinlänglich bekannt, daß der Schmelzpunkt im allgemeinen mit zunehmendem Anteil an langkettigen Fettsäuren bzw. mit abnehmendem Anteil an kurzkettigen oder ungesättigten Fettsäuren steigt. Die Eigenschaften eines Triglycerids werden auch durch die Stellung der verschiedenen Fettsäuregruppen innerhalb des Triglyceridmoleküls bestimmt.

Die Fettsäuren selbst sind überwiegend geradzahlige, geradkettige, aliphatische Monocarbonsäuren mit Kettenlängen von C₄ bis C₂₄. Der Schmelzpunkt einer Fettsäure fällt mit abnehmender Kettenlänge und zunehmender Anzahl von Doppelbindungen. Pflanzenöle sind bei Zimmertemperatur flüssig wegen ihres erheblichen Anteils an ungesättigten Fettsäuren.

Wenn das jeweilige Pflanzenöl als Ester der jeweiligen mehrfach ungesättigten Fettsäuren eingesetzt wird, sind grundsätzlich die Methylester von besonderem Interesse. Hier sind insbesondere Rapsfettsäuremethylester, Rizinolsäuremethylester, Ölsäuremethylester, Linolsäuremethylester, Laurinsäuremethylester zu nennen, wobei diese Aufzählung lediglich exemplarisch ist. Die Möglichkeiten sind mannigfaltig und die Auswahl sowie die gegebenenfalls weitere Umsetzung des pflanzlichen Öls oder seiner Bestandteile mit weiteren Fettsäuren ist für den Fachmann aufgrund seines allgemeinen Fachwissens ohne weiteres möglich.

Je nach Art des ausgewählten Pflanzenöls oder dessen Bestandteils verändert sich die Konsistenz des Mittels. Davon wird es abhängen, ob die Hautauflage mit dem Mittel durch Auftragen beschichtet oder aber imprägniert wird. Ozonisiertes Olivenöl mit einer Peroxidzahl von über 300 weist beispielsweise eine eher pastöse Konsistenz auf.

Die erfindungsgemäße medizinische Hautabdeckung steht aufgrund der guten Hautverträglichkeit und der umfassenden Wirksamkeit einer breiten Anwendung offen. So kann sie in Form einer Slip-Einlage eingesetzt werden.

Genauso ist sie in Form oder als Teil einer Windel oder eines desinfizierenden Unterlegvlieses einsetzbar.

Weiterhin bevorzugt ist die erfindungsgemäße, medizinische Hautabdeckung in Form eines Pflasters ausgebildet. Hier wird in bezug auf das Pflaster auf ein herkömmlich erhältliches Wundpflaster verwiesen, wie es z.B. aus der DE 6931110 T2 bekannt ist. Die so oder in einer anderen Weise ausgebildete medizinische Hautabdeckung kann zuschneidbar sein, so daß sie als Meterware zur Verfügung steht und jeweils nach Bedarf ein Streifen davon abgeschnitten werden kann. Wie bei einem herkömmlichen Pflaster gehört auch dazu, daß die Hautabdeckung mit einer Hautauflage versehen sein kann, die in verschiedenen Ausführungsformen unterschiedlicher Länge und/oder Breite vorliegt. Auf diese Weise kann die Hautabdeckung dem Anwendungszweck angepaßt werden. Die Behandlung eines entzündeten Pickels oder eines Furunkels wird eine andere Dimensionierung der Hautauflage erfordern als beispielsweise Grind oder Fußpilz.

Das Ozon weist üblicherweise aufgrund der Art seiner Herstellung in einem Ozongenerator Sauerstoff als Trägergas auf. Aufgrund dieses Sauerstoffgehalts ist es nicht erforderlich, dem ozonisierten Pflanzenöl und/oder dessen Bestandteilen, besonders der ozonisierten Ölsäure, ein Konservierungsmittel hinzuzufügen. Außerdem wird die Wundheilung durch den anwesenden Sauerstoff gefördert.

Zu einem gewissen Anteil werden auch die schon genannten Peroxide ausgebildet. Mit dem Gehalt an aktivem Sauerstoff, der in dieser peroxidischen Form gebunden ist, läßt sich die Wirksamkeit des ozonisierten Pflanzenöls bzw. die Wirksamkeit von dessen Bestandteilen nicht nur steigern, sondern dem hohen Anteil an Peroxiden, wie er in ozonisiertem Olivenöl zu finden ist, ist die desinfizierende Wirkung des Olivenöls und/oder ihrer Bestandteile zuzuschreiben.

Die Erfindung betrifft auch erfindungsgemäße Verwendungen der medizinischen Hautabdeckung zur Behandlung von entzündlichen Prozessen der Haut, vorzugsweise, aber nicht ausschließlich zur Behandlung von Furunkeln und Abszessen, Akne und Pilzinfektionen, "ulcus cruris" und diabetischem Fuß.

Als eine weitere bevorzugte Verwendung ist die Herstellung eines Zeckenpflasters zu nennen.

Im folgenden soll die Erfindung anhand von Ausführungsbeispielen, in Verbindung mit der beigefügten Zeichnung, näher erläutert werden.

Es zeigen:
- Fig. 1a:: Eine Aufsicht auf eine medizinische Hautabdeckung in rechteckiger Form,
- Fig. 1b:: eine Aufsicht auf eine medizinische Hautabdeckung in quadratischer Form,
- Fig.2:: eine Aufsicht auf eine medizinische Hautabdeckung in Form eines Streifens und
- Fig.3:: eine Aufsicht auf eine medizinische Hautabdeckung in Form eines Pflasters mit Wirkstoffdepot.

Die nachfolgend dargestellten und erläuterten Ausführungsformen und Anwendungen wurden grundsätzlich mit zwei verschiedenen antiphlogistischen und/oder antibakteriellen und/oder antiviralen und/oder antimykotischen Mitteln durchgeführt. Zum einen mit ozonisiertem Olivenöl und zum anderen mit ozonisierter Ölsäure. Zur Vermeidung von Wiederholungen wird im folgenden der einheitliche Begriff "wirksames ozonisiertes Mittel" verwendet, wenn Ausführungsformen, Anwendungen und Ergebnisse gleichermaßen für das ozonisierte Olivenöl wie die ozonisierte Ölsäure durchgeführt wurden und gelten.

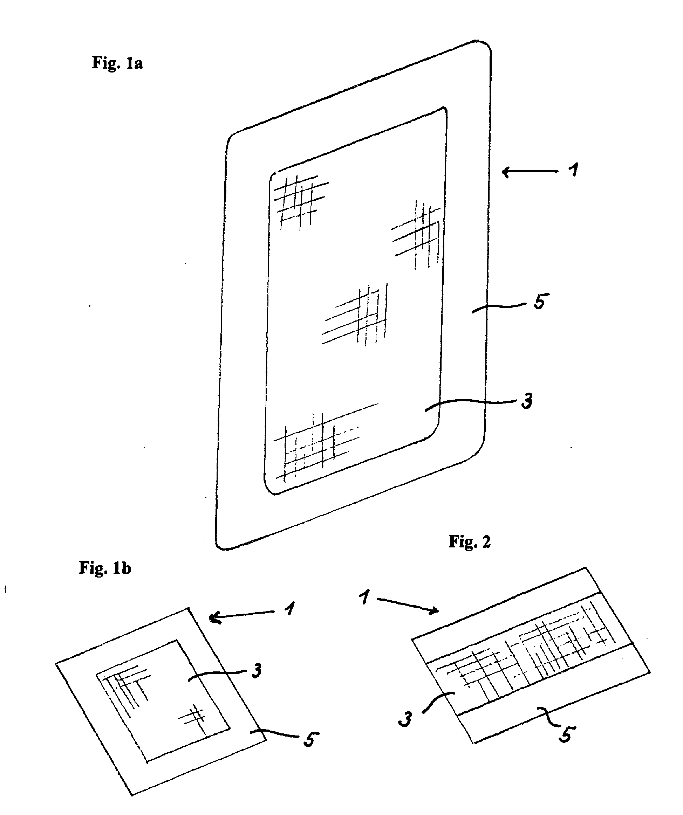

In Fig. 1 ist eine insgesamt mit der Bezugsziffer 1 bezeichnete medizinische Hautabdeckung dargestellt, die rechteckig ausgebildet ist und eine Hautauflage 3 und eine Deckschicht 5 aufweist. Die Deckschicht 5 ist vollflächig mit einer Klebeschicht bedeckt, die eine Haftkraft aufweist, wie sie auch bei herkömmlichen Pflastern üblich ist und typischerweise im Bereich von etwa 1 - 15 N/1 liegt.

Die Deckschicht 5 besteht im Ausführungsbeispiel aus einem textilen Flächengewebe in Form einer zugeschnittenen Trägergewebebahn. Alternativ kann die Deckschicht 5 z.B. aus einem Vlies bestehen, wie sie von der Firma Freudenberg erhältlich sind. Das Flächengewebe kann auch ein Gewirk oder ein Gestrick sein.

Die Größe der Deckschicht 5 ist völlig variabel wählbar und wurde für die Zwecke dieses Ausführungsbeispiels exemplarisch auf eine Kantenlänge von 5 x 3 cm eingestellt. Dabei wurden, wie aus Fig. 1 ersichtlich, die Ecken abgerundet, was auch nicht zwangsläufig erforderlich ist.

Auf die Deckschicht 5 wurde dann die Hautauflage 3 aufgebracht und über die Klebeschicht auf der Deckschicht 5 fixiert. Die Hautauflage 3 wurde hier jeweils 1,5 cm in Länge und Breite kleiner gewählt als die Deckschicht 5. Dadurch ergab sich ein für das Kleben auf der Haut eines Patienten zur Verfügung stehender allseitiger Klebestreifen 7 von 1,5 cm.

Vor dem Aufkleben wurde die Hautauflage 3 mit einem ozonisierten Olivenöl bestrichen, das eine pastöse Konsistenz aufwies.

Das Olivenöl wurde vor seiner Behandlung mit Ozon untersucht und wies eine Peroxidzahl von ca. 19 auf. Nach der Ozonisierung ergab sich eine Peroxidzahl von etwa 348. Unter der Peroxidzahl wird der Gehalt an aktivem Sauerstoff verstanden, der in peroxidischer Form gebunden ist. Peroxide sind grundsätzlich Folgeprodukt der Ozonide.

Das ozonisierte Olivenöl wurde als ein pastöses bis festes Produkt mit angenehm frischem Geruch erhalten, das folgende Zusammensetzung aufwies:

| | |
|---|---|
| - Ölsäure: | 5,6 Gew.-% |
| - Linolsäure: | 0,2 Gew.-% |
| - Palmitinsäure: | 7,0 Gew.-% |
| - Stearinsäure: | 2,7 Gew.-% |
| - ungesättigte Fettsäuren: | 8,3 Gew.-% |

Für folgende Bakterien [B] und Pilze [P] konnte der Nachweis erbracht werden, daß sie durch die mit dem ozonisierten Olivenöl bestrichenen Hautauflagen 3 der Hautabdeckungen 1 abgetötet werden:

| | |
|---|---|
| - Staphylococcus aureus | [B] |
| - Staphylococcus albus | [B] |
| - Staphylococcus hemolyticus | [B] |

| | |
|---|---|
| - Trichophyton mentagrophytes | [P] |
| - Trichophyton purpureum | [P] |
| - Microsporon audouini | [P] |
| - Microsporon lensonum | [P] |

Die erfindungsgemäßen Hautabdeckungen 1, welche die mit dem ozonisierten Olivenöl bestrichenen Hautauflagen 3 aufwiesen, wurden offen liegengelassen. Dabei trockneten die Hautauflagen 3 auch nach einem längeren offenen Liegenlassen nicht aus und wurden nicht rissig. Die Hautauflagen 3 haben sich bei insgesamt 15 Testpersonen durch eine überaus gute Verträglichkeit ausgezeichnet.

Neben den in Fig. 1 dargestellten rechteckigen Hautabdeckungen 1, die im übrigen genauso quadratisch ausgebildet sein können, wie es Fig. 1b zeigt, sind die erfindungsgemäßen medizinischen Hautabdeckungen 1 genauso in Form eines Streifens herstellbar. Dies zeigt Fig. 2. Wie bei einem Endlospflaster bzw. einem Pflaster in Streifenform ist auch hier die Deckschicht 5 in beliebiger Länge ausführbar. Für den Fachmann ist es ohne weiteres ersichtlich, daß die Breite fest gewählt ist, aber variabel dem Verwendungszweck angepaßt sein kann.

Ebenso ist es für eine handelsübliche Ausführungsform der erfindungsgemäßen Hautabdeckung 1 vorgesehen, daß diese auf der der Haut abgewandten Seite der Hautabdeckung 1 mit einer Schutzschicht, z.B. in Form einer Folie versehen wird, wie dies bei einem Pflaster auch üblich und bereits hinlänglich bekannt ist. Diese Schutzschicht ist in den Fig. nicht noch einmal näher dargestellt, da sie von Pflastern jeglicher Art bereits gut bekannt ist. An sich ist es selbstverständlich, eine medizinische Hautauflage, welche eine antiphlogistische und/oder antibakterielle und/oder antivirale und/oder antimykotische Wirkung entfalten soll, bis zum Anbringen auf dem zu behandelnden Hautbereich gut vor einer Verschmutzung zu schützen.

Nachfolgend werden Anwendungen der medizinischen Hautabdeckung 1 näher erläutert, wobei die Hautabdeckungen 1 jeweils die mit dem ozonisierten Olivenöl bestrichenen Hautauflagen 3 aufweisen.

In der gleichen Weise wie zuvor beschrieben, wurde eine medizinische Hautabdeckung hergestellt, die anstelle von ozonisiertem Olivenöl ozonisierte Ölsäure aufwies. Die mit der ozonisierten Ölsäure erzielten Ergebnisse sind vergleichbar mit den für das ozonisierte Olivenöl angegebenen Resultaten. Die nachfolgend erläuterten Anwendungen gelten ebenso für Versuche mit ozonisierter Ölsäure, weshalb hier - sofern erforderlich - die Formulierung "wirksames ozonisiertes Mittel" verwendet wird, wie weiter oben eingeführt.

### Anwendung als Aknepflaster

Für diese Anwendung wurde kein textiles Flächengewebe in Form einer zugeschnittenen Trägergewebebahn verwendet, wie dies eingangs beschrieben worden ist, sondern eine durchsichtige Hydrokolloidfolie als Trägermaterial. Die Größe und Form dieser Hydrokolloidfolie wurde an entsprechende Krankheitsherde bei durch Akne bedingte Hautentzündungen angepaßt. Entsprechend wurden kleine runde Zuschnitte, u.a. mit einem Durchmesser zwischen etwa 1,0 - 3,0 cm angefertigt, wobei diese Angaben lediglich exemplarisch für die Zwecke dieses Ausführungsbeispiels zu verstehen sind. Ebenso wurden kleine quadratische und rechteckige Pflasterformen der durchsichtigen Hydrokolloidfolie verwendet und die Ecken dabei leicht abgerundet.

Zur Prüfung der Wirksamkeit wurden die gram-positiven Bakterien "Propionibacterium acnes" herangezogen, die als solche zur natürlichen mikrobiellen Flora der Haut gehören, aber auch an Infektionen der Haut, wie Akne vulgaris, beteiligt sein können. Die Pflaster wurden täglich gewechselt. Es konnte mit beiden wirksamen ozonisierten Mitteln eine gute Wirkung gegen diese Bakterien nachgewiesen werden.

### Anwendung als Herpespflaster

Auch für diese Anwendung wurde kein textiles Flächengewebe in Form einer zugeschnittenen Trägergewebebahn verwendet, sondern eine durchsichtige Hydrokolloidfolie als Trägermaterial. Die Größe und Form dieser Hydrokolloidfolie wurde an Krankheitsherde angepasst, wie sie durch Herpesbläschen im Lippenbereich entstehen. Es ist bekannt, daß Viren, die Herpesviren, diese Herpesbläschen hervorrufen.

Die Pflaster wurden täglich gewechselt.

Überraschend und zunächst nicht erwartet war hier wie auch bei anderen Anwendungen der erfindungsgemäßen medizinischen Hautabdeckung, daß bei ihrer Anwendung im Bereich schmerzhafter Infektionen das Schmerzgefühl gelindert bis ganz ausgeschaltet werden konnte. Entsprechend konnte hier ein mit solchen Herpesinfektionen üblicherweise einhergehendes Schmerzgefühl nicht nur vermindert werden, sondern es klang ganz ab. Es konnte somit auch eine gute Wirkung der erfindungsgemäßen medizinischen Hautabdeckung mit beiden wirksamen ozonisierten Mitteln gegen Viren nachgewiesen werden. Gleichzeitig schützt das Hydrokolloidpflaster den von der Infektion betroffenen Hautbereich. Die Klebekraft des Pflasters wurde gerade so eingestellt, daß es gut wieder entfernt werden konnte, ohne daß dies als unangenehm empfunden wurde.

### Anwendung als Wundheilpflaster zur Behandlung von "ulcus cruris"

Die medizinische Hautabdeckung gemäß der vorliegenden Erfindung kann nach Wahl in ganz unterschiedlichen Größen konfektioniert werden.

Gemäß der hier beschriebenen Anwendung kommt sie auch in Größenordnungen zum Einsatz, die ein herkömmliches Pflaster überschreiten. Dabei wird sie hier als fertig beschichtete Wundabdeckung im Rahmen der Behandlung von "ulcus cruris"-Patienten bei einem Ulcus cruris venosum eingesetzt.

Die Heilung solcher Geschwüre ist bereits durch die mangelnde Durchblutung der entsprechenden Gewebepartien erschwert und wird zusätzlich durch die Ansiedlung von unterschiedlichsten Bakterien verhindert. Üblicherweise werden solche Geschwüre von teilweise heftigen Schmerzen begleitet. Sie stellen normalerweise nicht spontan abheilende Wunden dar, zumal sie bevorzugt in höherem Alter auftreten.

Die erfindungsgemäße medizinische Hautabdeckung schafft hier eine Wundabdeckung, die erstaunlicherweise in allen drei Problembereichen wirksam eingesetzt werden kann.

Zunächst ist hier die erstaunliche schmerzlindernde Wirkung der wirksamen ozonisierten Mittel hervorzuheben, mit denen die Hautauflagen jeweils bestrichen wurden. Bei dieser wie bei den weiteren, beschriebenen Anwendungen empfinden die behandelten Patienten eine deutliche Erleichterung im Schmerzempfinden, das von einem Nachlassen des Schmerzes bis zu einer Schmerzfreiheit beschrieben wurde.

Die Heilung der Wunde wird in zweifacher Weise befördert. Einerseits dient die Beschichtung mit den antibakteriell wirksamen ozonisierten Mitteln dazu, den Bakterienbefall deutlich zu reduzieren. Dadurch entsteht eine erhebliche Entlastung, die der Wundheilung zugute kommt.

Des weiteren ist es bereits bekannt und wird hier zunutze gemacht, daß Sauerstoff eine wesentliche Grundvoraussetzung für eine erfolgreiche Wundheilung ist. Hier wird insbesondere auf die Übersichtsarbeit von Herrn Priv.-Doz. Dr. med. Stefan Beckert et al., Chirurgische Universitätsklinik Tübingen, verwiesen.

Das Ozon weist aufgrund der Art seiner Herstellung in einem Ozongenerator Sauerstoff als Trägergas auf. Der auf diese Weise in den wirksamen ozonisierten Mitteln enthaltene Sauerstoff fördert daher die Wundheilung.

Da zu einem gewissen Anteil auch Peroxide ausgebildet werden, läßt sich die Wirksamkeit des ozonisierten Pflanzenöls wie der ozonisierten Ölsäure mit dem Gehalt an aktivem Sauerstoff, der in dieser peroxidischen Form gebunden ist, noch steigern.

Dies gilt nicht nur für die Behandlung von Ulcus cruris, sondern für die Wundheilung infolge der Anwendung der erfindungsgemäßen medizinische Hautabdeckung allgemein.

Die erzielten Behandlungserfolge waren sehr gut. Sie sind photographisch dokumentiert und gemessen. Es wurden Keimabstriche vor und nach der Behandlung vorgenommen, die Keimzahl ermittelt und dokumentiert.

### Anwendung als Wundheilpflaster zur Behandlung von "diabetischem Fuβ"

Im Zusammenhang mit Diabetes mellitus können ebenso "offene Beine" in Form von Ulcus cruris entstehen, die in gleicher Weise mit einer Wundauflage gemäß der vorliegenden Erfindung zu einer verbesserten Heilung gebracht werden können, wie das zuvor beschriebene Ulcus cruris venosum.

Häufig geht mit Diabetes mellitus auch ein sogenanntes diabetisches Fuβsyndrom einher, das schwierig zu heilen ist.

Patienten mit diabetischen Füßen wurden im Rahmen von Anwendungsbeobachtungen mit fertig beschichteten Wundauflagen in Form der erfindungsgemäßen medizinischen Hautabdeckung behandelt. Auch hier waren die erzielten Behandlungserfolge sehr gut. Sie sind photographisch dokumentiert und gemessen. Es wurden Keimabstriche vor und nach der Behandlung vorgenommen, die Keimzahl ermittelt und dokumentiert.

### Entzündungen des Nagelbetts

Von Entzündungen des Nagelbetts (Paronychie) ist es bekannt, daß sie häufig durch eine Infektion mit Staphylokokken verursacht werden. Dabei sind diese nicht zwangsläufig die Hauptursache. Oft ist der Nagelwall bereits durch Candida besiedelt. Die Therapie besteht üblicherweise in der Anwendung von Antimykotika.

Die Gefahr solcher Paronychien besteht darin, daß die Infektion in die Tiefe wandert und ein Panaritium entsteht, das in der Regel einen chirurgischen Eingriff erfordert.

Aufgrund der antimykotischen Wirkung der eingesetzten wirksamen ozonisierten Mittel konnte bei Entzündungen des Nagelbetts eine erstaunlich gute Wirkung der beiden ozonisierten Mittel bei der Behandlung solcher Entzündungen des Nagelbetts beobachtet werden.

Durchweg positiv wurde von den behandelten Patienten bewertet, daß ein durch die Entzündung bedingtes Schmerz- und/oder Druckempfinden an den befallenen Stellen ebenfalls nachließ bis ganz verschwand. Auf ein Antimykotikum konnte ganz verzichtet werden.

### Fuβ- und Nagelpilzerkrankungen

Fußpilzerkrankungen sind häufig und befallen in der Regel die Zehenzwischenräume und die Fußsohlen. Ausgelöst werden sie durch Fadenpilze.

Wird ein solcher Befall nicht behandelt, kommt es zu einer Abwehrschwächung der Haut, so daß es dann zu einer Streptokokkeninfektion und als Folge davon sogar zu einer Wundrose kommen kann.

Bei der Anwendung der medizinischen Hautabdeckung, welche mit den jeweiligen wirksamen ozonisierten Mitteln in der oben beschriebenen Weise versehen war, wurde der Fußpilz schon nach wenigen Anwendungen und innerhalb weniger Tage entfernt. Die medizinische Hautabdeckung wurde dabei täglich gewechselt.

### Zeckenpflaster

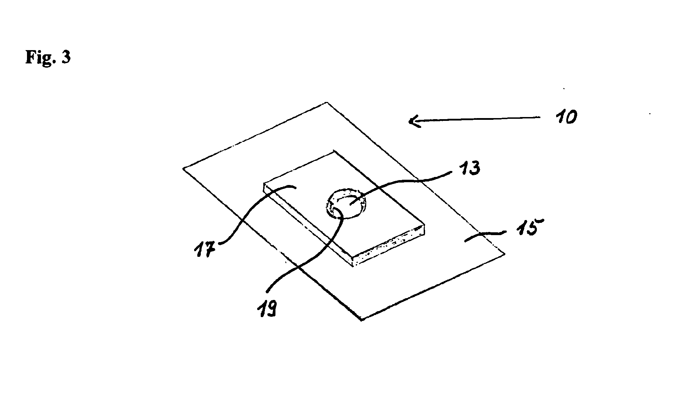

In Fig. 3 ist eine weitere Ausführungsform der medizinischen Hautabdeckung in Form eines Pflasters dargestellt. Das in Fig. 3 näher dargestellte Ausführungsbeispiel unterscheidet sich von der Ausführungsform nach den Fig. 1a, 1b im wesentlichen dadurch, daß der Wirkstoff in einem Depot gehalten wird. Entsprechend sind zu den Fig. 1a und 1b gleiche Merkmale mit denselben, jedoch um 10 erweiterten Bezugsziffern versehen.

Die in Fig. 3 dargestellte medizinische Hautabdeckung 10 weist eine in etwa quadratisch oder rechteckig ausgebildete Hautauflage 13 und eine Deckschicht 15 auf. Die Deckschicht 15 ist vollflächig mit einer Klebeschicht bedeckt, die wieder eine Haftkraft aufweist, wie sie auch bei herkömmlichen Pflastern üblich ist und typischerweise im Bereich von etwa 1 - 15 N/l liegt.

Die Deckschicht 15 besteht in diesem Ausführungsbeispiel aus einem Kunststoff. Alternativ wurde hier für die Deckschicht 15 ein transparenter, selbstklebender Kunststoff verwendet.

Für die Deckschicht 15 wurde in diesem Ausführungsbeispiel eine Kantenlänge von 6 x 3 cm gewählt, wobei die gegenüber der Breite größere Länge als Klebestreifen für eine gute Verklebung genutzt wird. Andere Kantenlängen sind möglich.

Auf die Deckschicht 15 wurde mittig und zentriert die Hautauflage 13 mit jeweils einer geringeren Kantenlänge aufgebracht und über die Klebeschicht der Deckschicht 15 auf dieser fixiert. In Abwandlung des ersten Ausführungsbeispiels wurde hier auf die mit der Hautauflage 13 versehene Deckschicht 15 zusätzlich eine Abdeckschicht 17 aufgebracht, die ebenfalls von der Klebeschicht der Deckschicht 15 gehalten ist. Bei dieser Ausführung sind somit drei Schichten vorhanden. Auf die Deckschicht 15 ist zunächst die Hautauflage 13 aufgebracht und mit der Deckschicht 15 verklebt. Darauf folgt dann die Abdeckschicht 17. Diese weist im Ausführungsbeispiel eine Größe von 3 x 3 cm auf. D.h. an der Schmalseite weist sie die gleiche Größe wie die Deckschicht 15 auf. Die Abdeckschicht 17 überragt mit ihrer Fläche die Hautauflage 13. Auf diese Weise kann ebenfalls mit der Deckschicht 15 verklebt werden. Dabei bleibt allseitig noch ein Teil der Deckschicht 15 frei, der zur Verklebung des Pflasters 10 mit der Umgebung dient, auf der das Pflaster 10 angebracht werden soll. Auf diese Weise kann die medizinische Hautabdeckung an stärker belasteten Stellen sicher angebracht werden.

In einer Abwandlung wird die Hautauflage 13 so auf die Abdeckschicht aufgebracht, daß nur jeweils in Längsrichtung ein Rand verbleibt, der verklebt werden kann.

Zentriert weist die Abdeckschicht 17 eine kreisrunde Aussparung 19 auf. Es wurde probeweise auch eine eckige Aussparung verwendet, die ebenso gute Ergebnisse erzielte. Bei einer etwa kreisrunden Aussparung 19 hat sich ein Durchmesser von ca. 1 cm als ausreichend erwiesen. Auch geringere oder größere Durchmesser können geeignet sein.

Die Hautauflage 13 wurde zum einen mit einer Paste bestrichen, welche im wesentlichen aus ozonisiertem Olivenöl besteht, und zum anderen mit der etwas dünnflüssigeren ozonisierten Ölsäure durch leichtes Tränken beschichtet.

Durch die Aussparung 19 gelangt ein definierter Bereich des jeweiligen wirksamen ozonisierten Mittels in Kontakt mit der Hautstelle, von der zuvor eine Zecke entfernt worden ist und kann dort seine antibakterielle und ggfls. auch antivirale Wirkung entfalten. Somit kann es gegen verschiedene Formen von Borreliose und gleichzeitig auch gegen die Frühsommerenzephalitis (FSME) wirken. Durch die Ränder der Aussparung 19 wird der Wirkstoff an der Stichstelle gehalten und kann nicht auslaufen.

Das ozonisierte Olivenöl in seiner pastösen bis festen Form weist eine Zusammensetzung auf, wie sie weiter oben wiedergegeben ist. Die ozonisierte Ölsäure ist im vergleich dazu etwas dünnflüssiger.

Die Hautverträglichkeit der auf diese Weise hergestellten Pflaster war im Vergleich zu den weiter oben beschriebenen Pflastern unverändert gut.

Auch das Pflaster 10 dieses Ausführungsbeispiels ist auf seiner das jeweilige wirksame ozonisierte Mittel aufweisenden Seite mit einer Schutzschicht in Form einer abziehbaren Folie versehen, die etwa mittig zweigeteilt sein kann, wie Pflaster sie üblicherweise aufweisen.

Solche medizinischen Pflaster werden in der Regel über einen Zeitraum von bis zu drei Tagen getragen. Danach sollten sie entfernt bzw. gewechselt werden, um das Mikroklima der Haut unterhalb des Pflasters nicht ungünstig zu verändern. Vorzugsweise sollten sie täglich gewechselt werden.

### Slip-Einlage und Binden

Die hier eingesetzte Slip-Einlage weist mehrere Schichten auf. Die als Hautauflage dienende Oberschicht entspricht der dem Körper zugewandten Seite, während die Deckschicht auf der dem Körper abgewandten Seite als Unterschicht bezeichnet wird. Dazwischen und somit im Innern der Slipeinlage befindet sich ein Saugkern. Dieser kann variiert werden, je nachdem, welche Aufnahmefähigkeit die Slipeinlage haben soll. Grundsätzlich besteht der Saugkern alternativ aus Zellulose- oder Kunststofffasern und weist im Ausführungsbeispiel noch einen Superabsorber auf.

Der Superabsorber ist quellfähig. Die aufgenommene Flüssigkeit wird in ein Gel verwandelt und im Saugkem eingeschlossen.

Das Oberflächenmaterial der Oberschicht ist aus einem weichen (Kunststoff-) Vlies gebildet. Zur schnellen Weiterleitung der aufzunehmenden Flüssigkeit befindet sich zwischen der Oberschicht und dem Saugkern noch eine Transferschicht.

Die Deck- bzw. Unterschicht weist eine Wäscheschutzfolie auf, die mit dem Saugkern verklebt ist. Nach außen ist auf der Unterschicht ein Klebestreifen als Klebeschicht angebracht, um die Slipeinlage z.B. an einem Slip zu befestigen.

Slip-Einlagen dieser Art und dieses Aufbaus sind grundsätzlich bekannt. Sie werden als medizinische Hautabdeckung eingesetzt, indem auf ihrer Oberschicht jeweils das wirksame ozonisierte Mittel dünn aufgetragen wird. Sein Geruch wird von den Versuchspersonen übereinstimmend jeweils als "medizinisch frisch" und angenehm bezeichnet. Der jeweilige dünne Auftrag wird nicht als unangenehm oder störend empfunden und ist gut hautverträglich. Reizungen konnten nicht festgestellt werden.

Mit diesen Slip-Einlagen können Keime u.a. aus der Gruppe der B- und D-Streptokokken abgetötet und daran gehindert werden, in den Uro-Genital-Trakt zu wandern. Diese Keime werden somit daran gehindert, im Uro-Genital-Trakt Infektionen auszulösen.

Besonders wertvoll ist diese Anwendung auch für die Behandlung im Rahmen von Schwangerschaften, da ebenso wirkungsvoll Infektionen des ungeborenen Kindes verhindert werden können.

Die gleiche Anwendung gilt auch für Binden. Diese weisen ebenso wie die Slip-Einlagen grundsätzlich um den Saugkern eine wasserabweisende Deckschicht auf der dem Körper abgewandten Seite als Unterschicht und eine als Hautauflage dienende Oberschicht auf der dem Körper zugewandten Seite auf, welche mit dem jeweiligen wirksamen ozonisierten Mittel beschichtet ist.

### Windel

Die erfindungsgemässe Verwendung soll hier exemplarisch an Einwegwindeln beschrieben werden. Diese in ihrem Aufbau an sich bekannten Windeln bestehen aus einem im Ausführungsbeispiel mit einem Superabsorber angereicherten Saugkern aus Zellstoff, der von einem (Kunststoff-) Vlies umgeben ist. Als Kunststoff kann hier Polyester genannt werden. Damit die eindringende Flüssigkeit im Inneren gehalten werden kann, wird eine Windel von einer Kunststofffolie umgeben.

Bei der in dieser Weise aufgebauten Windel wird die Hautauflage im weitesten Sinne als der Bereich angesehen, in dem die Windel an dem Intimbereich anliegt. Dort ist das wirksame ozonisierte Mittel jeweils dünn aufgetragen. Als Deckschicht wird im weitesten Sinne die nach außen weisende Windeloberfläche bezeichnet, welche Klebstreifen zur Fixierung der Windel aufweist.

Bei der Säuglingspflege wie bei Inkontinenz, hier insbesondere, aber nicht ausschließlich in der Altenpflege, soll mit der Beschichtung durch das wirksame ozonisierte Mittel im Intimbereich einer Windeldermatitis oder anderen Infektionen vorgebeugt oder diese sollen wirksam bekämpft werden, sofern sie bereits vorhanden sind. Grundsätzlich gelten die gleichen Beobachtungen, wie weiter oben zu den Slip-Einlagen beschrieben.

Es kann jeweils nach individuellem Bedarf die kombinierte antiphlogistische, antibakterielle, antivirale und/oder antimykotische Wirkung des jeweiligen wirksamen ozonisierten Mittels ausgenutzt werden.

### Unterlegvlies

Im Rahmen der Unterlegvliese sind zwei verschiedene Anwendungsbereiche zu unterscheiden, die im folgenden erläutert werden sollen.

Bei der einen Anwendung steht das Entfernen und/oder Vermeiden von Keimen an Infusionsnadeln, Kathetern und dergleichen im Vordergrund.

Insbesondere wenn diese für eine längere Liegedauer bestimmt sind, besteht das Problem, das Keime vorhanden sind, die sich vermehren und in das Körperinnere eindringen können.

Um dies wirksam zu verhindern, wurden konfektionierte, steril verpackte, saugfähige Vliespads eingesetzt, die auf ihrer einen, im Anwendungsfall der jeweiligen Haut zugewandten Seite mit einer dünnen Beschichtung aus dem wirksamen ozonisierten Mittel versehen sind. Diese Pads weisen seitlich eine Öffnung auf, im Ausführungsbeispiel in Form eines Schlitzes, zum schnellen und problemlosen Wechseln bei z.B. einem liegenden Katheter. Auf diese Weise kann der Bereich um den Katheter-Eintritt in den Körper oder der Bereich um eine Nadel keimfrei gehalten werden.

Wie festzustellen war, steigt die desinfizierende Wirkung des jeweiligen wirksamen ozonisierten Mittels langsam und kontinuierlich über Stunden an, so daß auf diese Weise eine vergleichsweise lang anhaltende desinfizierende Wirkung zu beobachten ist.

Dieses Unterleg-Vlies sollte bei liegender Nadel oder bei liegendem Katheter regelmäßig gewechselt werden. Ein täglicher Wechsel ist dabei bevorzugt.

Eine weitere Form des Unterlegvlieses betrifft die Pflege von Dekubituspatienten. Unter Dekubitus bzw. Dekubitalgeschwüren versteht man dabei das insbesondere bei längerer Bettlägerigkeit auftretende Wundliegen.

Kann diesem nicht durch andere Maßnahmen wirksam oder ausreichend entgegengewirkt werden, bildet es einen schwer auszuschaltenden Ort für Keime verschiedenster Art. Problematisch ist dabei, daß diese nicht nur lokal zu Infektionen führen, sondern je nach Schwere des Dekubitalgeschwürs durch Streuung von Eiterherden in die Blutbahn gelangen und unter Umständen eine Lungenentzündung auslösen können.

Hier steht nun mit der erfindungsgemäßen medizinischen Hautabdeckung ein wirksames keimtötendes Mittel zur Verfügung, das die betroffenen Stellen vor weiterer Infektion schützt.

Zusätzlich wurde eine schmerz- und schnell den Wundgeruch lindernde Wirkung festgestellt.

## Patentansprüche

1. Medizinische Hautabdeckung zur Behandlung und Verhinderung von Infektionen der Haut, mit einer Hautauflage (3) und einer Deckschicht (5), wobei die Hautauflage mit einem antiphlogistischen und/oder antibakteriellen und/oder antiviralen und/oder antimykotischen Mittel versehen ist, das ozonisiertes Pflanzenöl und/oder dessen Bestandteile umfaßt.

2. Medizinische Hautabdeckung nach Anspruch 1, **dadurch gekennzeichnet, daß** die Deckschicht (5) auf der der Haut zu- oder abgewandten Seite zumindest teilweise eine Klebeschicht aufweist.

3. Medizinische Hautabdeckung nach Anspruch 2, **dadurch gekennzeichnet, daß** die Deckschicht (5) auf der der Haut zugewandten Seite zumindest teilweise eine Klebeschicht aufweist, und daß die Hautauflage (3) an der Klebeschicht befestigt ist.

4. Medizinische Hautabdeckung nach einem der Ansprüche 1 bis 3, **dadurch gekennzeichnet, daß** die Deckschicht (5) eine größere Fläche aufweist als die Hautauflage (3).

5. Medizinische Hautabdeckung nach einem der Ansprüche 1 bis 4, **dadurch gekennzeichnet, daß** das Pflanzenöl ausgewählt ist aus Rapsöl, Sonnenblumenkernöl, Sojaöl, Rizinusöl, Olivenöl, Leinöl, Kokosöl, Palmöl, Teebaumöl, zumindest einem Bestandteil, Derivat und/oder Mischungen davon.

6. Medizinische Hautabdeckung nach Anspruch 5, **dadurch gekennzeichnet, daß** das Derivat ein Ester ist, vorzugsweise ein Glycerinester einer mehrfach ungesättigten Fettsäure, und daß der Bestandteil des Pflanzenöls Fettsäure, bevorzugt Ölsäure ist.

7. Medizinische Hautabdeckung nach einem der Ansprüche 1 bis 6 in Form einer Slip-Einlage.

8. Medizinische Hautabdeckung nach einem der Ansprüche 1 bis 6 in Form oder als Teil einer Windel.

9. Medizinische Hautabdeckung nach einem der Ansprüche 1 bis 6 in Form eines desinfizierenden Unterlegvlieses.

10. Medizinische Hautabdeckung nach einem der Ansprüche 1 bis 6, **dadurch gekennzeichnet, daß** sie in Form eines Pflasters ausgebildet ist.

11. Medizinische Hautabdeckung nach Anspruch 10, **dadurch gekennzeichnet, daß** sie zuschneidbar ausgebildet ist.

12. Medizinische Hautabdeckung nach einem der Ansprüche 1 bis 11, **dadurch gekennzeichnet, daß** das Ozon Sauerstoff als Trägergas aufweist.

13. Verwendung der medizinischen Hautabdeckung nach einem der Ansprüche 1 bis 12 zur Herstellung eines Präparats für die Behandlung von entzündlichen Prozessen der Haut.

14. Verwendung nach Anspruch 13 zur Herstellung eines Präparats für die Behandlung von Furunkeln und Abszessen, Akne und Pilzinfektionen, "ulcus cruris" und diabetischem Fuß.

15. Verwendung der medizinischen Hautabdeckung nach einem der Ansprüche 10 bis 12 zur Herstellung eines Zeckenpflasters.
